# EUROPEAN PATENT APPLICATION

(11) **EP 2 374 416 A2**
(43) Date of publication of application: **12.10.2011**
(21) Application number: 11250290.1
(22) Date of filing: 11.03.2011
(51) Int. Cl.: A61B 17/04, A61B 17/062, A61B 17/00, A61B 17/06, A61B 17/29

(54) **Deployment system for surgical suture**

(30) Priority: 12.03.2010 US 722860
(71) Applicant: Tyco Healthcare Group LP, New Haven, CT 06511 (US)
(72) Inventor: Bogart, Michael, Milford, CT 06460 (US); Krehel, Gregg, Newtown, CT 06470 (US); Fishbein, Christopher, Wolcott, CT 06716 (US); Pribanic, Russell, Roxbury CT 06783 (US); Kirsch, David, Madison, CT 06443 (US); Horton, Kenneth W., Jr., South Glastonbury, CT 06073 (US); Kosa, Timothy D., Hamden, CT 06514 (US); Maiorino, Nicholas, Branford, CT 06405 (US); Buchter, Mark S., Ansonia, CT 06401 (US); Hadba, Ahmad Robert, Middlefield, CT 06455 (US); Hodgkinson, Gerald, Guilford, CT 06437 (US); Bowns, William R., Ansonia, CT 06401 (US)
(74) Representative: Soames, Candida Jane

(57) **Abstract**

A suture deployment system is disclosed including a surgical needle having a flexible suture fixedly attached thereto and a target suture releasably secured to the flexible suture. Either the flexible suture or the target suture may be made of a shape memory polymer. A collar is positioned between the flexible suture and the target suture and is fixedly secured to the flexible suture. The target suture is releasably secured to the collar is such that the target suture may detach from the collar once a surgical procedure has been completed. The surgical needle is a double pointed surgical needle specifically configured to be manipulated by a surgical suturing apparatus.

## Description

### CROSS-REFERENCE TO RELATED APPLICATIONS

The present application is a continuation-in-part of U.S. Patent Application Serial No. 12/415,288, filed on March 31, 2009, which claims the benefit of and priority to U.S. Provisional Application Serial No. 61/123,900, filed on April 11, 2008, the entire disclosures of each of which are incorporated by reference herein.

The present application also claims the benefit of and priority to International Application Serial No. PCT/US07/21482, filed on October 4, 2007, International Application Serial No. PCT/US07/21412, filed on October 5, 2007, International Application Serial No. PCT/US07/21447, filed on October 5, 2007, International Application Serial No. PCT/US07/21449, filed on October 5, 2007, International Application Serial No. PCT/US07/21457, filed on October 5, 2007, International Application Serial No. PCT/US07/21466, filed on October 5, 2007, International Application Serial No. PCT/US07/21495, filed on October 5, 2007, International Application Serial No. PCT/US07/21496, filed on October 5, 2007, International Application Serial No. PCT/US07/21506, filed on October 5, 2007, the entire content of each of which being incorporated herein by reference.

### BACKGROUND

The present disclosure relates to a deployment system for insertion of a surgical suture into the body of a patient. More particularly, the present disclosure relates to a deployment system for use in inserting a length of a relatively stiff suture into the body of a patient laparoscopically.

Various surgical procedures often require the use of relatively stiff sutures to repair or reconnect tough tissues. Stiff sutures may be required in open surgical procedures where ligaments or tendons are being reconnected or may be required in endoscopic and/or laparoscopic surgical procedures where the stiff suture is required to be passed down through a narrow cannula along with an associated surgical instrument.

Attachment of the stiff sutures directly to a surgical needle may limit the ability to manipulate the surgical needle through the tough tissues without risk of tearing the tissues with the stiff suture or damaging the stiff suture itself by excessive bending of the suture. Further, direct attachment of the surgical needle to the stiff suture may limit the ability to advance the surgical needle and stiff suture through cannula structure to access the interior of a body cavity.

Therefore, it is desirable to provide a system for inserting a surgical needle and a stiff suture through tissues without risk of damage to the tissues and bending of the stiff suture. It would further be desirable to provide a system for deploying a surgical needle and a stiff suture through a cannula to dispose the surgical needle and stiff suture within a body cavity without risk of bending or breaking the suture.

### SUMMARY

The present disclosure provides suture deployment systems and methods for making and utilizing same. In embodiments, a suture deployment of the present disclosure may include a surgical needle; a flexible suture having a distal end and a proximal end, the distal end of the flexible suture being attached to the surgical needle; and a target suture having a distal end and a proximal end, wherein the distal end of the target suture is secured to the proximal end of the flexible suture, and wherein at least one of the flexible suture and the target suture include a shape memory polymer.

In other embodiments, a suture deployment system of the present disclosure may include a surgical needle having a body portion and a tissue penetrating tip formed at each end of the body portion; a multifilament suture having a distal end and a proximal end, the distal end of the multifilament suture being secured within the suture hole in the body portion of the surgical needle; a hollow collar having a distal end and a proximal end, the proximal end of the multifilament suture being within the distal end of the hollow collar; and a monofilament target suture having a distal end and a proximal end, wherein the distal end of the monofilament target suture is releasably secured within the proximal end of the hollow collar, and wherein at least one of the multifilament suture and monofilament target suture include a shape memory material.

In embodiments, the barbed suture may be a compound barbed suture including an elongated body; and at least one barb extending from the elongated body and defining an inner surface, the inner surface including a first portion disposed at a first orientation relative to a longitudinal axis of the elongated body and a second portion disposed at a second orientation relative to the longitudinal axis.

### DESCRIPTION OF THE DRAWINGS

An embodiment of the presently disclosed suture deployment system is disclosed herein with reference to the drawings, wherein:

FIG. 1 is a perspective view of a suture deployment system with parts separated;

FIG. 2 is an enlarged area of detail view of FIG. 1;

FIG. 2A is a plan view of a segment of a barbed suture of the suture deployment system of FIG. 1;

FIG. 3 is a perspective view of the assembled suture deployment system of FIG. 1;

FIG. 4 is a perspective view of the suture deployment system of FIG. 1 and a surgical suturing apparatus for use with the suture deployment system;

FIG. 4A is a perspective view of a distal end of a surgical suturing apparatus according to a further embodiment for use with the suture deployment system of FIG. 1;

FIG. 4B is a perspective view of a distal end of a surgical suturing apparatus according to yet a further embodiment for use with the suture deployment system of FIG. 1;

FIG. 5 is an enlarged side view, partially shown in section, of the surgical suturing apparatus of FIG. 4 advancing the suture deployment system through a cannula;

FIG. 6 is an enlarged side view, partially shown in section, of the surgical suturing apparatus positioning the suture deployment system about a pair of tissues;

FIG. 7 is an enlarged side view, partially shown in section, of the surgical suturing apparatus driving a needle of the suture deployment system through the pair of tissues;

FIG. 8 is an enlarged side view, partially shown in section, of the surgical suturing apparatus pulling an intermediate suture, a sleeve and a target suture of the suture deployment system through the tissues; and

FIG. 9 is an enlarged side view, partially shown in section, of the surgical suturing apparatus pulling the target suture through the tissues.

### DETAILED DESCRIPTION OF EMBODIMENTS

An embodiment of the presently disclosed suture deployment system will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

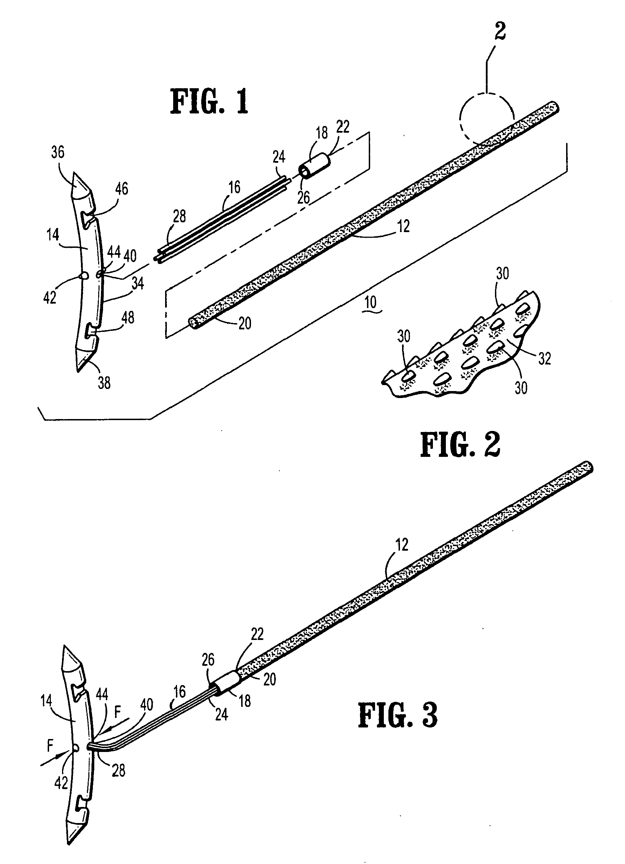

Referring to FIG. 1, there is disclosed a suture deployment system 10 for use in carrying, and manipulating, a relatively stiff target suture 12 of suture deployment system 10 into the body of a patient. Suture deployment system 10 generally includes target suture 12 and a surgical needle 14 for piercing tissue so as to position target suture 12 therethrough. A relatively flexible suture 16 is positioned between surgical needle 14 and target suture 12 and allows surgical needle 14 to be manipulated within a body cavity and through tissue without causing undue bending of target suture 16 as described in more detail below. Additionally, flexible suture 16 enables target suture 12 to be connected to surgical needle 14 and inserted through a cannula without danger of bending or breaking target suture 12. A hollow ferrule or collar 18 is provided to releasably connect flexible suture 16 to target suture 12 and allow target suture 12 to remain in tissue after the remainder of suture deployment system 10 is removed from the body of the patient.

Specifically, a distal end 20 of target suture 12 is releasably held within a proximal end 22 of collar 18. A proximal end 24 of flexible suture 16 is fixedly secured within a distal end 26 of collar 18. Likewise, a distal end 28 of flexible suture 16 is fixedly secured to surgical needle 14 in a manner described in more detail below.

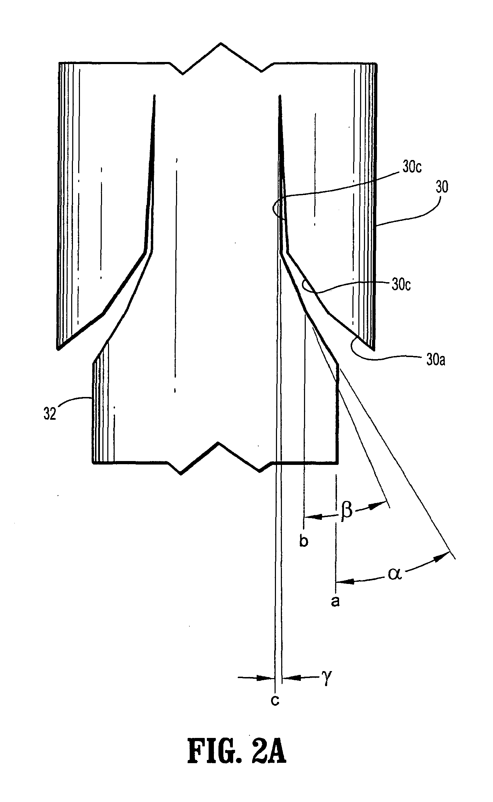

Referring for the moment to FIG. 2, target suture 12 is a stiff suture of the type used in connection with the repair of relatively tough tissues where suture strength and not bending or wrapping of the suture material is most important. Examples of such applications may include, for example, ligament or tendon repair and reconnection, etc. Target suture 12 may be a "barbed suture" having a series of anchoring projections or barbs 30 formed on an outer surface 32 thereof. Barbs 30 facilitate anchoring target suture 12 within the relatively tough tissues.

Referring back to FIG. 1, in contrast to target suture 12, flexible suture 16 is relatively flaccid which allows surgical needle 14 to be manipulated without putting bending stresses on target suture 12. In one embodiment, flexible suture 16 is a relatively short length of multifilament suture of a type typically known in the art. As noted above, collar 18 is provided to releasably connect target suture 12 to flexible suture 16. Collar 18 may be formed from a variety of biocompatible materials, such as, for example, polymeric materials, stainless steel, etc.

Sutures of the present disclosure may be absorbable or non-absorbable. It should be understood that combinations of filaments made from different materials (e.g. natural and synthetic, or bioabsorbable and non-bioabsorbable materials) may be used to make the present sutures.

Suitable synthetic absorbable materials include polymers such as those made from lactide, glycolide, caprolactone, valerolactone, carbonates (e.g., trimethylene carbonate, tetramethylene carbonate), dioxanones (e.g., 1,4-dioxanone) δ-valerolactone, 1,dioxepanones (e.g., 1,4-dioxepan-2-one and 1,5-dioxepan-2-one), ethylene glycol, ethylene oxide, esteramides, γ-hydroxyvalerate, β-hydroxypropionate, alpha-hydroxy acid, hydroxybuterates, orthoesters, hydroxy alkanoates, tyrosine carbonates, polyimide carbonates, polyimino carbonates such as poly (bisphenol A-iminocarbonate) and poly (hydroquinone-iminocarbonate), and polymer drugs (e.g., polydiflunisol, polyaspirin, and protein therapeutics) and copolymers and the like and combinations thereof. Suitable natural absorbable polymers include collagen, cellulose, gut, and the like, and combinations of these. In embodiments, glycolide and lactide based polyesters, including copolymers of lactide and glycolide may be used.

Suitable non-absorbable materials which may be used to form the sutures disclosed herein include non-absorbable natural materials such as cotton, silk, rubber, and the like, and combinations of these. Suitable non-absorbable synthetic materials include monomers and polymers derived from materials such as nylons, polyolefins such as polypropylene and polyethylene, ultra high molecular weight polyethylene (UHMWPE), polyamides, polyesters such as poly butylene terephthalate (PBT), poly trimethylene terephthalate (PTT), poly ethylene terephthalate (PET), polyaryletherketone, polyvinylidene difluoride (PVDF), acrylic, polyamides, aramids, fluropolymers, polybutesters, silicones, and the like, and polymer blends, copolymers thereof and combinations with degradable polymers. Polypropylene can also be utilized to form the suture. The polypropylene can be isotactic polypropylene or a mixture of isotactic and syndiotactic or atactic polypropylene. Additionally, non-absorbable synthetic and natural polymers and monomers may be combined with each other and may also be combined with various absorbable polymers and monomers to create fibers and filaments for the present sutures.

As used herein, the terms "fibers", "filaments" and "yarns" each may be used to construct the sutures in whole or in part. The term "fibers," in this context, are generally used to designate natural or synthetic structures that have a length approximately 3 orders of magnitude greater than their diameter or width. The term "filaments" are typically used to describe "fibers" of indefinite or extreme length, and "yarns" as a generic term for a continuous strand of twisted or untwisted "fibers" or "filaments" in a form suitable for knitting, weaving, braiding or otherwise intertwining.

Flexible sutures 16 of the present disclosure may be multifilament (e.g. braided) and target sutures 12 may be monofilamant. Methods for making sutures from these suitable materials are within the purview of those skilled in the art (e.g. extrusion and molding). The filaments may be combined to create a multifilament suture using any technique within the purview of one skilled in the art such as commingling, twisting, braiding, weaving, entangling, and knitting. For example, filaments may simply be combined to form a yarn or they may be braided. In another example, filaments may be combined to form a yarn and then those multifilament yarns may be braided. Those skilled in the art reading this disclosure will envision other ways in which filaments may be combined. Fibers may also be combined to produce a non-woven multifilament large diameter suture. In certain embodiments, a multifilament structure useful in forming an anchoring suture according to the present disclosure may be produced by braiding. The braiding can be done by any method within the purview of those skilled in the art. Furthermore, the anchoring suture may include portions which are monofilament and portions which are multifilament. In some embodiments, the proximal end of the elongate body may be a multifilament and the looped portion (loop portion described below) may be a monofilament.

In embodiments, suitable materials which may be utilized to form the sutures in accordance with the present disclosure include homopolymers, copolymers, and/or blends possessing glycolic acid, lactic acid, glycolide, lactide, dioxanone, trimethylene caprolactone, and various combinations of the foregoing. For example, in some embodiments, a copolymer of glycolide and trimethylene carbonate may be utilized. Methods for forming such copolymers are within the purview of those skilled in the art and include, for example, the methods disclosed in U.S. Patent Nos. 4,300,565 and 5,324,307, the entire disclosures of each or which are incorporated by reference herein. Suitable copolymers of glycolide and trimethylene carbonate may possess glycolide in amounts from about 60% to about 75% by weight of the copolymer, in embodiments, from about 65% to about 70% by weight of the copolymer, with the trimethylene carbonate being present in amounts from about 25% to about 40% by weight of the copolymer, in embodiments, from about 30% to about 35% by weight of the copolymer.

Other suitable materials include copolymers of lactide and glycolide, with lactide present in an amount from about 6% to about 12% by weight of the copolymer and glycolide being present in amounts from about 88% to about 94% by weight of the copolymer. In some embodiments, lactide is present from about 7% to about 11% by weight of the copolymer with glycolide being present in amounts from about 89% to about 98% by weight of the copolymer. In some other embodiments, lactide is present in an amount of about 9% by weight of the copolymer with the glycolide being present in an amount of about 91% by weight of the copolymer.

In embodiments, suitable materials for forming sutures according to the present disclosure include, in embodiments, copolymers of glycolide, dioxanone, and trimethylene carbonate. Such materials may include, for example, copolymers possessing glycolide in amounts from about 55% to about 65% by weight of the copolymer, in embodiments, from about 58% to about 62% by weight of the copolymer, in some embodiments, about 60% by weight of the copolymer; dioxanone in amounts from about 10% to about 18% by weight of the copolymer, in embodiments, from about 12% to about 16% by weight of the copolymer, in some embodiments about 14% by weight of the copolymer; and trimethylene carbonate in amounts from about 17% to about 35% by weight of the copolymer, in embodiments, from about 22% to about 30% by weight of the copolymer, in some embodiments, about 26% by weight of the copolymer.

Other suitable materials include a copolymer of glycolide, lactide, trimethylene carbonate, and ε-caprolactone may be utilized to form sutures in accordance with the present disclosure. Such materials may include, for example, a random copolymer possessing caprolactone in amounts from about 14% to about 20% by weight of the copolymer, in embodiments, from about 16% to about 18% by weight of the copolymer, in some embodiments, about 17% by weight of the copolymer; lactide in amounts from about 4% to about 10% by weight of the copolymer, in embodiments, from about 6% to about 8% by weight of the copolymer, in some embodiments about 7% by weight of the copolymer; trimethylene carbonate in amounts from about 4% to about 10% by weight of the copolymer, in embodiments from about 6% to about 8% by weight of the copolymer, in some embodiments about 7% by weight of the copolymer; and glycolide in amounts from about 60% to about 78% by weight of the copolymer, in embodiments, from about 66% to about 72% by weight of the copolymer, in some embodiments about 69% by weight of the copolymer.

In certain embodiments, the sutures in whole or in part (e.g. anchors/barbs) may be constructed using shape memory polymeric materials which are capable of adopting a shape in vivo suitable for adhering tissue or affixing a surgical device, such as a mesh, to tissue. Shape memory polymeric materials which may be utilized to form a suture of the present disclosure possess a permanent shape and a temporary shape. In embodiments, the temporary shape is of a configuration which enhances the ability for the surgeon to introduce a suture into a patient's body. The permanent shape, which is assumed in vivo upon application of energy, such as heat or light, is of a configuration which enhances the retention of the suture in tissue and/or adhesion of a surgical device to tissue.

Shape memory polymers are a class of polymers that, when formed into an object such as a suture, can be temporarily deformed by mechanical force and then caused to revert back to an original shape when stimulated by energy. Shape memory polymers exhibit shape memory properties by virtue of at least two phase separated microdomains in their microstructure. The first domain is composed of hard, covalently cross-linked or otherwise chain motion-limiting structures, which act as anchors to retain the object's original shape. The second domain is a switchable soft structure, which can be deformed and then fixed to obtain a secondary or temporary shape.

In the case of heat stimulated shape memory polymers, a transition temperature (T_{Trans}) exists at which the shape change occurs during heating. The shape memory polymers can thus be tailored by altering material properties at the molecular level and by varying processing parameters. An object's primary shape may be formed with heat and pressure at a temperature at which the soft domains are flexible and the hard domains are not fully formed. The object may then be cooled so that the hard domains are more fully formed and the soft domains become rigid. The secondary or temporary shape can be formed by mechanically deforming the object, which is most readily accomplished at a temperature approaching or above T_{Trans}. Mechanical stresses introduced into the object are then locked into place by cooling the object to temperatures below T_{Trans}, so that the soft segments solidify to a rigid state. Once the object is heated to T>T_{Trans}, the soft segments soften and relax back to their original configuration and the object returns to its primary shape, sometimes referred to herein, as its permanent shape. The temperature at which a shape memory material reverts to its permanent shape may be referred to, in embodiments, as its permanent temperature (Tₚₑᵣₘ).

Polymers possessing shape memory properties which may be used to construct sutures disclosed herein include, for example, synthetic materials, natural materials (e.g., biological) and combinations thereof, which may be biodegradable and/or non-biodegradable. As used herein, the term "biodegradable" includes both bioabsorbable and bioresorbable materials. By biodegradable, it is meant that the materials decompose, or lose structural integrity under body conditions (e.g., enzymatic degradation, hydrolysis) or are broken down (physically or chemically) under physiologic conditions in the body (e.g., dissolution) such that the degradation products are excretable or absorbable by the body.

Suitable non-degradable materials which may possess shape memory properties include, but are not limited to, polyolefins such as polyethylene (including ultra high molecular weight polyethylene) and polypropylene including atactic, isotactic, syndiotactic, and blends thereof; polyethylene glycols; polyethylene oxides; ultra high molecular weight polyethylene; copolymers of polyethylene and polypropylene; polyisobutylene and ethylene-alpha olefin copolymers; fluorinated polyolefins such as fluoroethylenes, fluoropropylenes, fluoroPEGs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polytetramethylene ether glycol; polybutesters, including copolymers of butylene terephthalate and polytetramethylene ether glycol; 1,4-butanediol; polyurethanes; acrylic polymers; methacrylics; vinyl halide polymers and copolymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins such as ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof. Additionally, non-biodegradable polymers and monomers may be combined with each other.

Suitable bioabsorbable polymers which may possess shape memory properties include, but are not limited to, aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof.

Suitable aliphatic polyesters may include, but are not limited to, homopolymers and copolymers of lactide (including lactic acid, D-,L- and meso lactide); glycolide (including glycolic acid); epsilon-caprolactone; p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof.

Other suitable biodegradable polymers include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof. Collagen as used herein includes natural collagen such as animal derived collagen, gelatinized collagen, or synthetic collagen such as human or bacterial recombinant collagen.

Additionally, synthetically modified natural polymers such as cellulose and polysaccharide derivatives, including alkyl celluloses, hydroxyalkyl celluloses, cellulose ethers, cellulose esters, nitrocelluloses, and chitosan may be utilized. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose (CMC), cellulose triacetate, and cellulose sulfate sodium salt. These may be collectively referred to herein, in embodiments, as "celluloses."

In embodiments, combinations of both degradable and non-degradable materials, including those having shape memory characteristics, may be utilized.

In embodiments, the shape memory polymer may be a copolymer of two components with different thermal characteristics, such as oligo (epsilon-caprolactone) dimethacrylates and butyl acrylates, including poly(epsilon-caprolactone) dimethacrylate-poly (n-butyl acrylate), or a diol ester and an ether-ester diol such as oligo (epsilon caprolactone) diol/oligo (p-dioxanone) diol copolymers. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory Polymers-Biodegradable Sutures," Materials World, Vol. 10, no. 7, pp. 29-30 (July 2002), the entire disclosure of which is incorporated by reference herein.

In other embodiments, blends of bioabsorbable materials may be utilized including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

Other examples of suitable shape memory polymers and means for forming permanent and temporary shapes therewith are set forth in Lendlein et al., "Shape memory polymers as stimuli-sensitive implant materials," Clinical Hemorheology and Microcirculation, 32 (2005) 105-116, Lendlein et al., "Biodegradable, Elastic Shape memory Polymers for Potential Biomedical Applications," Science, Vol. 269 (2002) 1673-1676, and Lendlein et al., "Shape-Memory Polymers," Angew. Chem. Int. Ed., 41 (2002) 2035-2057, the entire disclosures of each of which are incorporated by reference herein.

Table 1 below further illustrates compositions which demonstrate shape memory effects. The block copolymers of each composition are in annealed wire format, the proposed soft and hard segments, and the glass transition temperature (Tg), having been measured by differential scanning calorimetry which is equal to T_{Trans}.

**TABLE 1**

| Composition (mol%) | Soft Domain | Hard Domain | T_{g} (T_{Trans}) [°C] |
|---|---|---|---|
| 15% Polydioxanone 85% Poly (L-lactide) | Polydioxanone and Amorphous Polylactide | Crystalline Polylactide | 54 |
| 20% Polydioxanone 80% Poly (L-lactide) | Polydioxanone and Amorphous Polylactide | Crystalline Polylactide | 45 |
| 15% Trimethylene Carbonate 85% Poly (L-lactide) | Trimethylene Carbonate and Amorphous Polylactide | Crystalline Polylactide | 54 |
| 20% Trimethylene Carbonate 80% Poly (L-lactide) | Trimethylene Carbonate and Amorphous Polylactide | Crystalline Polylactide | 55 |

The copolymers in Table 1 may undergo a partial shift when approaching T_{g} and T_{Trans} may be depressed when the materials are in aqueous solution. Since these polymers degrade by water absorption and bulk hydrolysis, water molecules entering the polymer matrices may act as plasticizers, causing the soft segments to soften at lower temperatures than in dry air. Thus, polymers exhibiting T_{Trans} depression in aqueous solution may maintain a temporary shape through temperature excursions in the dry state, such as during shipping and storage, and shape shift to its permanent shape at body temperatures upon implantation.

Thus, in embodiments, the shape memory polymer may include a block copolymer of polydioxanone and polylactide with the polydioxanone present in an amount from about 5 mol% to about 20 mol% of the copolymer, in embodiments from about 15 mol% to about 19 mol% of the copolymer, and the polylactide present in an amount from about 80 mol% to about 95 mol% of the copolymer, in embodiments from about 81 mol% to about 85 mol% of the copolymer. In other embodiments, the shape memory polymer may include a block copolymer of trimethylene carbonate and polylactide, with the trimethylene carbonate present in an amount from about 5 mol% to about 20 mol% of the copolymer, in embodiments from about 15 mol% to about 19 mol% of the copolymer, and the polylactide may be present in an amount from about 80 mol% to about 95 mol% of the copolymer, in embodiments from about 81 mol% to about 85 mol% of the copolymer.

It is envisioned that T_{Trans} may be tailored by changing block segment molar ratios, polymer molecular weight, and time allowed for hard segment formation. In embodiments, T_{Trans} may be tailored by blending various amounts of low molecular weight oligomers of the soft segment domain into the copolymer. Such oligomers may segregate to soft domains and act as plasticizers to cause a downward shift in T_{Trans}.

Additionally, the copolymers forming the sutures of the present disclosure may include emulsifying agents, solubilizing agents, wetting agents, taste modifying agents, plasticizers, active agents, water soluble inert fillers, preservatives, buffering agents, coloring agents, and stabilizers. Addition of a plasticizer to the formulation can improve flexibility. The plasticizer or mixture of plasticizers may be polyethylene glycol, glycerol, sorbitol, sucrose, corn syrup, fructose, dioctyl-sodium sulfosuccinate, triethyl citrate, tributyl citrate, 1,2-propylenglycol, mono-, di- or triacetates of glycerol, or natural gums.

In some embodiments, crystalline degradable salts or minerals may be added to the block copolymer compositions to create polymer composites which may improve shape memory properties. An example of such a composite using polylactide homopolymer and crystalline hydroxyapatite is described in Zheng et al., "Shape memory properties of poly (D,L-lactide/hydroxyapatite composites," Biomaterials, 27 (2006) 4288-4295, the entire disclosure of which are incorporated by reference herein.

Other shape memory materials, including shape memory metals (e.g. steel and degradable manganese), and metal alloys such as Nitinol, may also be used to form the sutures of the present disclosure.

In embodiments, a molding process may be utilized to produce the sutures of the present disclosure. Plastic molding methods are within the purview of those skilled in the art and include, but are not limited to, melt molding, solution molding, and the like. Injection molding, extrusion molding, compression molding and other methods can also be used as the melt molding technique. Once placed in the mold with the proper dimensions and configuration, the polymeric material used to form the suture may be heated to a suitable temperature, such as the permanent temperature (Tₚₑᵣₘ) which may, in embodiments, be the melting temperature of the shape memory polymeric material utilized to form the suture. Heating of the suture may be at suitable temperatures including, for example, from about 40°C to about 180°C, in embodiments from about 80°C to about 150°C, for a period of time of from about 2 minutes to about 60 minutes, in embodiments from about 15 minutes to about 20 minutes, to obtain the permanent shape and dimensions.

The temperature for deformation treatment of the anchoring member molded with a previously memorized shape is one that makes possible ready deformation without producing cracks and should not exceed the temperature adopted for the shape memorization (e.g., Tpₑᵣₘ). Deformation treatment at a temperature exceeding that for the original shape memorization may cause the object to memorize/program a new deformed shape.

After the suture with the desired shape has been formed, the suture may be deformed above T_{Trans} to obtain an alternate, temporary shape.

Suitable temperatures for deformation will vary depending on the shape memory polymer utilized, but generally may be above the transition temperature of the polymer (Tₜᵣₐₙₛ), but below the Tₚₑᵣₘ. In embodiments, the shape memory polymer may be cooled from its Tₚₑᵣₘ to a lower temperature which remains above the Tₜᵣₐₙₛ and deformed, in embodiments by hand and/or mechanical means. In other embodiments, the suture may be deformed at room temperature (about 20° C to about 25° C) to obtain its temporary shape, although the temperature may differ depending upon the particular polymer employed. The suture may then be cooled to a temperature below the Tₜᵣₐₙₛ of the material utilized to form the sutures, at which time the suture of the present disclosure is ready for use. As the Tₜᵣₐₙₛ is usually greater than room temperature, in embodiments cooling to room temperature may be sufficient to lock in the temporary shape.

There are no particular limitations on the manner in which the deformation can be achieved. Deformation can be achieved either by hand or by means of a suitable device selected to provide the desired temporary configuration to the suture.

In order to keep the shape of the suture in its temporary shape, the shape memory sutures of the present disclosure should be stored at a temperature which will not cause a transition to the primary shape. In embodiments, the shape memory suture may be stored in a refrigerator.

In embodiments, the shape memory polymeric materials of the present disclosure may be compressed or expanded into temporary forms that are smaller or larger in diameter than their permanent shape.

The sutures thus prepared recover their permanent shape upon application of energy, such as on heating, either by placement in a patient's body, or by the addition of exogenous heat at a prescribed temperature, in embodiments above the Tₜᵣₐₙₛ of the shape memory polymer utilized. As the sutures of the present disclosure are utilized in a living body, heating with body heat (about 37° C) is possible. In such a case, the temperature for shape programming should be as low as possible and the recovery of the permanent shape may occur fairly slowly. In embodiments, recovery of the permanent shape may occur from about 1 second to about 5 seconds after insertion into tissue.

However, in some embodiments a higher shape memory temperature may be desirable in order to make the shape recover at a slightly higher temperature than body temperature. Thus, in some cases, releasing the suture from deformation to recover the permanent shape can be achieved by heating. On heating at a temperature of from about 30° C to about 50° C, in embodiments from about 39° C to about 43° C, the temporary shape may be released and the permanent shape recovered. The higher the temperature above T_{Trans} for heating, the shorter the time required for recovery of the permanent shape. The means for this heating is not limited. Heating can be accomplished by using a gas or liquid heating medium, heating devices, ultrasonic waves, electrical induction, and the like. Of course, in an application involving a living body, care must be taken to utilize a heating temperature which will not cause bums. Examples of liquid heating media include physiological saline solution, alcohol, combinations thereof, and the like.

Similarly, in other embodiments, electrically active polymers, also known as electroactive polymers, which can alter their configuration upon application of electricity, may be utilized to fashion sutures in accordance with the present disclosure. Suitable examples of electroactive polymers include poly(aniline), substituted poly(aniline)s, polycarbazoles, substituted polycarbazoles, polyindoles, poly(pyrrole)s, substituted poly(pyrrole)s, poly(thiophene)s, substituted poly(thiophene)s, poly(acetylene)s, poly(ethylene dioxythiophene)s, poly(ethylenedioxypyrrole)s, poly(p-phenylene vinylene)s, and the like, or combinations including at least one of the foregoing electroactive polymers. Blends or copolymers or composites of the foregoing electroactive polymers may also be used.

Similar to the change in shape which a shape memory material may undergo upon the application of energy, such as heat, in embodiments an electroactive polymer may undergo a change in shape upon the application of electricity from a low voltage electrical source (such as a battery). Suitable amounts of electricity which may be applied to effect such change will vary with the electroactive polymer utilized, but can be from about 5 volts to about 30 volts, in embodiments from about 10 volts to about 20 volts. The application of electricity will result in the suture constructed of the electroactive polymer changing its shape into a fastening configuration.

While an electroactive polymer does not have the same permanent shape and temporary shape as those terms are described above with respect to shape memory polymers, as used herein the term "permanent shape" as applied to an electroactive polymer means, in embodiments, the shape the electroactive polymer adopts upon the application of electricity, and the term "temporary shape" as applied to an electroactive polymer means, in embodiments, the shape of the electroactive polymer adopts in the absence of electricity.

Filaments used for forming sutures of the present disclosure may be formed using any technique within the purview of those skilled in the art, such as, for example, extrusion, molding and/or solvent casting.

In embodiments, the suture of the present disclosure may include a yarn made of more than one filament, which may contain multiple filaments of the same or different materials.

As used herein, the terms "fibers", "filaments" and "yarns" each may be used to construct sutures, in whole or in part. The term "fibers," in this context, are generally used to designate natural or synthetic structures that have a length approximately 3 orders of magnitude greater than their diameter or width. The term "filaments" are typically used to describe "'fibers" of indefinite or extreme length, and "yarns" as a generic term for a continuous strand of twisted or untwisted "fibers" or "filaments" in a form suitable for knitting, weaving, braiding or otherwise intertwining.

In embodiments, sutures of the present disclosure may possess a core/sheath configuration, fibers may possess a core/sheath configuration, yarns may possess a core/sheath configuration, or both. Any material described herein, including the shape memory materials described above, may be utilized to form the core, the sheath, or both.

Sutures of the present disclosure may be monofilament or multifilament (e.g. braided). Methods for making sutures from these suitable materials are within the purview of those skilled in the art (e.g. extrusion and molding). The filaments may be combined to create a multifilament suture using any technique within the purview of one skilled in the art such as commingling, twisting, braiding, weaving, entangling, and knitting. For example, filaments may be combined to form a yarn or they may be braided. In another example, filaments may be combined to form a yarn and then those multifilament yarns may be braided. Those skilled in the art reading this disclosure will envision other ways in which filaments may be combined. Fibers may also be combined to produce a non-woven multifilament large diameter suture. In certain embodiments, a multifilament structure useful in forming a suture according to the present disclosure may be produced by braiding. The braiding can be done by any method within the purview of those skilled in the art. For example, braid constructions for sutures and other medical devices are described in U.S. Patent Nos. 5,019,093; 5,059,213; 5,133,738; 5,181,923; 5,226,912; 5,261,886; 5,306,289; 5,318,575; 5,370,031; 5,383,387; 5,662,682; 5,667,528; and 6,203,564; the entire disclosures of each of which are incorporated by reference herein. Furthermore, the suture may include portions which are monofilament and portions which are multifilament.

Once the suture is constructed, it can be sterilized by any means within the purview of those skilled in the art.

Therapeutic agents may be utilized with the sutures in accordance with the present disclosure. Therapeutic agents include, but are not limited to, drugs, amino acids, peptides, polypeptides, proteins, polysaccharides, muteins, immunoglobulins, antibodies, cytokines (e.g., lymphokines, monokines, chemokines), blood clotting factors, hemopoietic factors, interleukins (1 through 18), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone, luteinizing hormone releasing factor), vaccines (e.g., tumoral, bacterial and viral antigens); somatostatin; antigens; blood coagulation factors; growth factors (e.g., nerve growth factor, insulin-like growth factor); bone morphogenic proteins, TGF-B, protein inhibitors, protein antagonists, and protein agonists; nucleic acids, such as antisense molecules, DNA, RNA, RNAi; oligonucleotides; polynucleotides; cells, viruses, and ribozymes.

In embodiments, the therapeutic agent may include at least one of the following drugs, including combinations and alternative forms of the drugs such as alternative salt forms, free acid form, free base forms, pro-drugs and hydrates: analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloide, morphine, oxycodone, codeine, dihydrocodeine bitartrate, pentazocine, hydrocodone bitartrate, levorphanol, diflunisal, trolamine salicylate, nalbuphine hydrochloride, mefenamic acid, butorphanol, choline salicylate, butalbital, phenyltoloxamine citrate, diphenhydramine citrate, methotrimeprazine, cinnamedrine hydrochloride, and meprobamate); antiasthmatics (e.g., ketotifen and traxanox); antibiotics (e.g., neomycin, streptomycin, chloramphenicol, cephalosporin, ampicillin, penicillin, tetracycline, and ciprofloxacin); antidepressants (e.g., nefopam, oxypertine, doxepin, amoxapine, trazodone, amitriptyline, maprotiline, phenelzine, desipramine, nortriptyline, tranylcypromine, fluoxetine, doxepin, imipramine, imipramine pamoate, isocarboxazid, trimipramine, and protriptyline); antidiabetics (e.g., biguanides and sulfonylurea derivatives); antifungal agents (e.g., griseofulvin, ketoconazole, itraconizole, amphotericin B, nystatin, and candicidin); antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine); anti-inflammatories (e.g., (non-steroidal) indomethacin, ketoprofen, flurbiprofen, naproxen, ibuprofen, ramifenazone, piroxicam, (steroidal) cortisone, dexamethasone, fluazacort, celecoxib, rofecoxib, hydrocortisone, prednisolone, and prednisone); antineoplastics (e.g., cyclophosphamide, actinomycin, bleomycin, dactinomycin, daunorubicin, doxorubicin, epirubicin, mitomycin, methotrexate, fluorouracil, gemcitabine, carboplatin, carmustine (BCNU), methyl-CCNU, cisplatin, etoposide, camptothecin and derivatives thereof, phenesterine, paclitaxel and derivatives thereof, docetaxel and derivatives thereof, vinblastine, vincristine, goserelin, leuprolide, tamoxifen, interferon alfa, retinoic acid (ATRA), nitrogen mustard alkylating agents, and piposulfan); antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene); immunosuppressive agents (e.g., cyclosporine, azathioprine, mizoribine, and FK506 (tacrolimus)); antimigraine agents (e.g., ergotamine, propanolol, isometheptene mucate, and dichloralphenazone); sedatives/hypnotics (e.g., barbiturates such as pentobarbital, pentobarbital, and secobarbital; and benzodiazapines such as flurazepam hydrochloride, triazolam, and midazolam); antianginal agents (e.g., beta-adrenergic blockers; calcium channel blockers such as nifedipine, and diltiazem; and nitrates such as nitroglycerin, isosorbide dinitrate, pentearythritol tetranitrate, and erythrityl tetranitrate); antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine); antimanic agents (e.g., lithium carbonate); antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, quinidine polygalacturonate, flecainide acetate, tocainide, and lidocaine); antiarthritic agents (e.g., phenylbutazone, sulindac, penicillanine, salsalate, piroxicam, azathioprine, indomethacin, meclofenamate, gold sodium thiomalate, ketoprofen, auranofin, aurothioglucose, and tolmetin sodium); antigout agents (e.g., colchicine, and allopurinol); anticoagulants (e.g., heparin, heparin sodium, and warfarin sodium); thrombolytic agents (e.g., urokinase, streptokinase, and alteplase); antifibrinolytic agents (e.g., aminocaproic acid); hemorheologic agents (e.g., pentoxifylline); antiplatelet agents (e.g., aspirin); anticonvulsants (e.g., valproic acid, divalproex sodium, phenytoin, phenytoin sodium, clonazepam, primidone, phenobarbitol, carbamazepine, amobarbital sodium, methsuximide, metharbital, mephobarbital, mephenytoin, phensuximide, paramethadione, ethotoin, phenacemide, secobarbitol sodium, clorazepate dipotassium, and trimethadione); antiparkinson agents (e.g., ethosuximide); antihistamines/antipruritics (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorpheniramine maleate, methdilazine, and); agents useful for calcium regulation (e.g., calcitonin, and parathyroid hormone); antibacterial agents (e.g., amikacin sulfate, aztreonam, chloramphenicol, chloramphenicol palirtate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate); antiviral agents (e.g., interferon alpha, beta or gamma, zidovudine, amantadine hydrochloride, ribavirin, and acyclovir); antimicrobials (e.g., cephalosporins such as cefazolin sodium, cephradine, cefaclor, cephapirin sodium, ceftizoxime sodium, cefoperazone sodium, cefotetan disodium, cefuroxime e azotil, cefotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium; penicillins such as ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium; erythromycins such as erythromycin ethylsuccinate, erythromycin, erythromycin estolate, erythromycin lactobionate, erythromycin stearate, and erythromycin ethylsuccinate; and tetracyclines such as tetracycline hydrochloride, doxycycline hyclate, and minocycline hydrochloride, azithromycin, clarithromycin); anti-infectives (e.g., GM-CSF); bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate; anticholinergic agents such as ipratropium bromide; xanthines such as aminophylline, dyphylline, metaproterenol sulfate, and aminophylline; mast cell stabilizers such as cromolyn sodium; inhalant corticosteroids such as beclomethasone dipropionate (BDP), and beclomethasone dipropionate monohydrate; salbutamol; ipratropium bromide; budesonide; ketotifen; salmeterol; xinafoate; terbutaline sulfate; triamcinolone; theophylline; nedocromil sodium; metaproterenol sulfate; albuterol; flunisolide; fluticasone proprionate; steroidal compounds and hormones (e.g., androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens; progestins such as methoxyprogesterone acetate, and norethindrone acetate; corticosteroids such as triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate; and thyroid hormones such as levothyroxine sodium); hypoglycemic agents (e.g., human insulin, purified beef insulin, purified pork insulin, glyburide, chlorpropamide, glipizide, tolbutamide, and tolazamide); hypolipidemic agents (e.g., clofibrate, dextrothyroxine sodium, probucol, pravastitin, atorvastatin, lovastatin, and niacin); proteins (e.g., DNase, alginase, superoxide dismutase, and lipase); nucleic acids (e.g., sense or anti-sense nucleic acids encoding any therapeutically useful protein, including any of the proteins described herein); agents useful for erythropoiesis stimulation (e.g., erythropoietin); antiulcer/antireflux agents (e.g., famotidine, cimetidine, and ranitidine hydrochloride); antinauseants/antiemetics (e.g., meclizine hydrochloride, nabilone, prochlorperazine, dimenhydrinate, promethazine hydrochloride, thiethylperazine, and scopolamine); as well as other drugs useful in the compositions and methods described herein include mitotane, halonitrosoureas, anthrocyclines, ellipticine, ceftriaxone, ketoconazole, ceftazidime, oxaprozin, albuterol, valacyclovir, urofollitropin, famciclovir, flutamide, enalapril, mefformin, itraconazole, buspirone, gabapentin, fosinopril, tramadol, acarbose, lorazepan, follitropin, glipizide, omeprazole, fluoxetine, lisinopril, tramsdol, levofloxacin, zafirlukast, interferon, growth hormone, interleukin, erythropoietin, granulocyte stimulating factor, nizatidine, bupropion, perindopril, erbumine, adenosine, alendronate, alprostadil, benazepril, betaxolol, bleomycin sulfate, dexfenfluramine, diltiazem, fentanyl, flecainid, gemcitabine, glatiramer acetate, granisetron, lamivudine, mangafodipir trisodium, mesalamine, metoprolol fumarate, metronidazole, miglitol, moexipril, monteleukast, octreotide acetate, olopatadine, paricalcitol, somatropin, sumatriptan succinate, tacrine, verapamil, nabumetone, trovafloxacin, dolasetron, zidovudine, finasteride, tobramycin, isradipine, tolcapone, enoxaparin, fluconazole, lansoprazole, terbinafine, pamidronate, didanosine, diclofenac, cisapride, venlafaxine, troglitazone, fluvastatin, losartan, imiglucerase, donepezil, olanzapine, valsartan, fexofenadine, calcitonin, and ipratropium bromide. In some embodiments, the drug may be water soluble. In some embodiments, the drug may not be water soluble.

Additionally, the sutures may include biologically acceptable additives such as plasticizers, antioxidants, dyes, dilutants, therapeutic agents, and the like and combinations thereof, which can be coated on the filaments or fibers, or impregnated into the fibers or filaments (e.g. during compounding or extrusion) used to form the suture of the present disclosure.

Various compositions and materials may also be applied to the sutures or included in the filaments or fibers to improve mechanical properties such as handling and knot strength or to deliver therapeutic agents. Suitable coating materials include any materials conventionally applied to sutures. For example, suitable materials include fatty acid esters which may be combined with the metal salt of a fatty acid in the coating composition. Such esters include, for example, calcium stearate, stearoyl lactylate esters, palmityl lactylate esters, oleyl lactylate esters such as calcium, magnesium, aluminum, barium, or zinc stearoyl lactylate, calcium, magnesium, aluminum, barium, or zinc palmityl lactylate; calcium, magnesium, aluminum, barium, or zinc oleyl lactylate; and the like, and combinations of these, with calcium stearate and calcium stearoyl-2-lactylate (such as the calcium stearoyl-2-lactylate commercially available under the trade name VERV from American Ingredients Co., Kansas City, Mo.) being preferred. When desirable, the fatty acid ester may be combined with a solvent. Suitable solvents include polar and non-polar solvents including but not limited to alcohols, such as, methanol, ethanol, propanol; chlorinated hydrocarbons such as methylene chloride, chloroform, 1, 2-dichloroethane; and aliphatic hydrocarbons such as hexane, heptene, ethyl acetate; and the like and combinations of these.

In embodiments, the sutures may be combined with and/or coated with suitable materials including polyalkylene oxides such as polyethylene oxide, polypropylene oxide, polyethylene glycol (PEG), polypropylene glycol, copolymers thereof, and the like, including those having acrylate groups such as acrylate PEGs, and acrylate PEG/PPG copolymers. Such combinations may include blends or copolymers with polyalkylene oxide oligomers or polymers or other non-toxic surfactants. The resulting composition may possess antimicrobial properties due to the presence of the copolymers described above. In other embodiments, the sutures may be combined with silicone acrylates. Coatings may be applied to the individual filaments or to the entire suture at any time prior to sterilization techniques. Coatings can be applied to the filaments using any technique within the purview of those skilled in the art.

Additionally, the sutures may incorporate various pharmaceuticals and therapeutic agents. Therapeutic agents and drugs may be applied to the sutures and/or construct materials by methods within the purview of those skilled in the art, including but not limited to dipping, spraying, brushing, vapor deposition, coextrusion, capillary wicking, film casting, molding and the like, and combinations of these. Additionally, solvents may be used to incorporate various agents into the anchoring suture. Suitable solvent include those listed above.

Methods for combining these therapeutic agents with compositions of the present disclosure are within the purview of those skilled in the art and include, but are not limited to mixing, blending, dipping, spraying, wicking, solvent evaporating and the like.

As mentioned above and as seen in FIG. 2, target suture 12 may be a "barbed suture" having a series of anchoring projections or barbs 30 formed on an outer surface 32 thereof. Barbs 30 facilitate anchoring target suture 12 within the relatively tough tissues.

The barbs can be arranged in any suitable pattern along a length thereof including helical, linear, or randomly spaced with respect to longitudinal axis "A". The number, configuration, spacing and surface area of the barbs can vary depending upon the tissue in which the suture is used, as well as the composition and geometry of the material utilized to form the suture. For example, if the wound closure device is intended to be used in fatty tissue, which is relatively soft, the barbs may be longer and spaced further apart to enable to suture to grip the soft tissue. The barbs can be arranged in various directions at various angles or a single barb may include more than one angle, such as a compound barb.

In an alternate embodiment, as seen in FIG. 2A, the sutures may include a compound barb having an inner surface including a first angle, disposed at a first orientation relative to a longitudinal axis "A" thereof and a second angle having a second inner surface, disposed at a second orientation relative to a longitudinal axis "B" thereof.

As seen in FIG. 2A, compound barbs 30 having first, second and third portions 12a-c are generally formed by cutting into the surface of elongated body 14. In embodiments, each of the first, second, and third portions 12a-c may be cut at first, second and third angles α, β, and γ relative to longitudinal axes a, b, and c respectively of elongated body 14 which are parallel to a central longitudinal axis 'D', wherein the second angle β is less than the first angle α, and the third angle γ is less than the second angle β. Compound barb 12 may include a first portion 12a which is formed by cutting into elongated body 14 at a first angle α of from about 0 degrees to about 90 degrees relative to longitudinal axis "a", in other embodiments, the first angle α ranges from about 30 degrees to about 50 degrees relative to longitudinal axis "a", a second portion 12b which is formed by cutting into elongated body 14 at a second angle β of from about 0 degrees to about 90 degrees relative to the longitudinal axis "b", in other embodiments, the second angle β ranges from about 2 degrees to about 25 degrees relative to the longitudinal axis "b", and optionally a third portion 12c which is formed by cutting into elongated body 14 at a third angle γ of from about 0 degrees to about 90 degrees relative to longitudinal axis "c", in other embodiments, the third angle γ ranges from about 2 degrees to about 50 degrees relative to longitudinal axis "c". Reference may be made to U.S. Provisional Application Serial No. 61/029,964, filed on February 20, 2008, entitled "Compound Barb Medical Device and Method," the entire content of which is incorporated herein by reference, for a detailed discussion of various geometries of compound barbs and the like.

The suture may optionally include a third orientation different from the first and second orientation. In an embodiment, the first, second and third orientations are each disposed at different angles with respect to the longitudinal axis. In some embodiments, the suture may include a staggered arrangement of large or small barbs. In other embodiments, the sutures may have a random configuration of both large and small barbs.

The surface area of the plurality of barbs can also vary. For example, fuller-tipped barbs can be made of varying sizes designed for specific surgical applications. When joining fat and relatively soft tissues, larger barbs may be desired, whereas smaller barbs may be more suitable for collagen-dense tissues. In some embodiments, a combination of large and small barbs within the same structure may be beneficial, for example when a fiber is used in tissue repair with differing layer structures. Use of the combination of large and small anchors with the same fiber wherein barb sizes are customized for each tissue layer will ensure maximum holding properties.

Surgical needle 14 is provided to pierce tissue and draw flexible suture 16 and target suture 12 through relatively tough tissue. Surgical needle 14 takes the form of any of the various known surgical needles used for carrying and threading suture material through tissue. In the disclosed embodiment, surgical needle 14 is double pointed needle specifically configured for use with a surgical suturing apparatus (described below) capable of driving surgical needle 14 through tough tissue. Surgical needle 14 generally includes a curved body portion 34 having first and second end points 36 and 38, respectively. Body portion 34 includes a central suture hole 44 for receipt of distal end 28 of flexible suture 16. A pair of crimping bumps 42 and 44 are provided adjacent suture hole 40 for securing flexible suture 16 within suture hole 40 as described in more detail hereinbelow. Engagement slots 46 and 48 are provided adjacent pointed ends 36 and 38. Engagement slots 46 and 48 are configured to alternately receive engagement structure associated with the surgical suturing apparatus, described hereinbelow, in order to pass surgical needle 14 back-and-forth between jaws of the surgical suturing apparatus. Exemplary of embodiments of surgical needle 14 are disclosed in U. S. Patent No. 5,569,301 to Granger et al., the entire disclosure of which is incorporated by reference herein.

Referring now to FIGS. 1 and 3, in order to assemble suture deployment system 10, proximal end 24 of flexible suture 16 is inserted within distal end 26 of collar 18 and fixedly secured therein by various known methods, such as, for example, gluing, welding, crimping, etc. Distal end 20 of target suture 12 is inserted within proximal end 22 of collar 18 and secured therein in a manner which will allow distal end 20 to separate from collar 18 upon the application of sufficient force to collar 18. This may be accomplished by utilizing weak adhesives, partial crimping of collar 18 about distal end 20 of target suture 12, or simply a friction fit of distal end 20 within proximal end 22 of collar 18. It should be noted that the securement of distal end 26 of collar 18 about proximal end 24 of flexible suture 16 is substantially stronger than the securement of distal end 20 of target suture 12 within proximal end 22 of collar 18. Thus, upon the application of sufficient tension on flexible suture 16, flexible suture 16 will remain attached to collar 18 while target suture 12 releasably detaches from collar 18.

In order to secure distal end 28 of flexible suture 16 to surgical needle 14, distal end 28 is inserted in suture hole 40 formed in body portion 34 of surgical needle 14. Thereafter, a crimping force is applied in the directions of arrows F to compress or crimp bumps 42 and 44 causing suture hole 40 to close or crimp about distal end 28 of flexible suture 16 (see U.S. Patent No. 5,569,301 to Granger et al.).

While a collar 18 has been described and illustrated for connecting target suture 12 and flexible suture 16 to one another, other methods are contemplated and included in the present disclosure, including and not limited to the use of an overmold extending over and between target suture 12 and flexible suture 16, the fusing of target suture 12 and flexible suture 16 to one another, and the like.

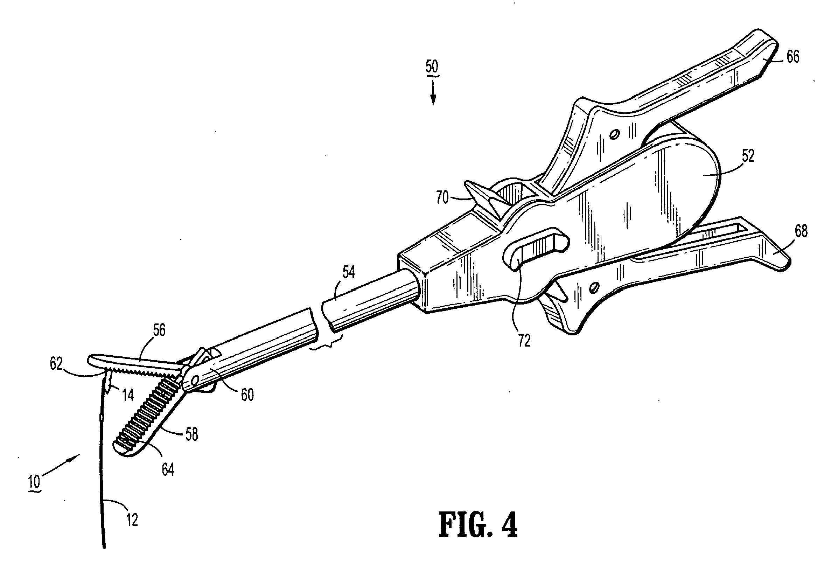

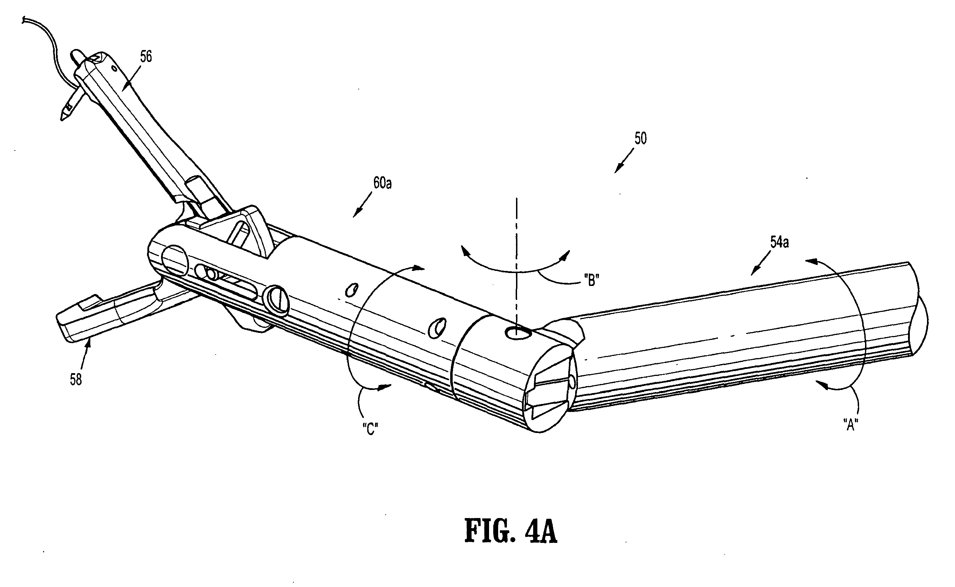

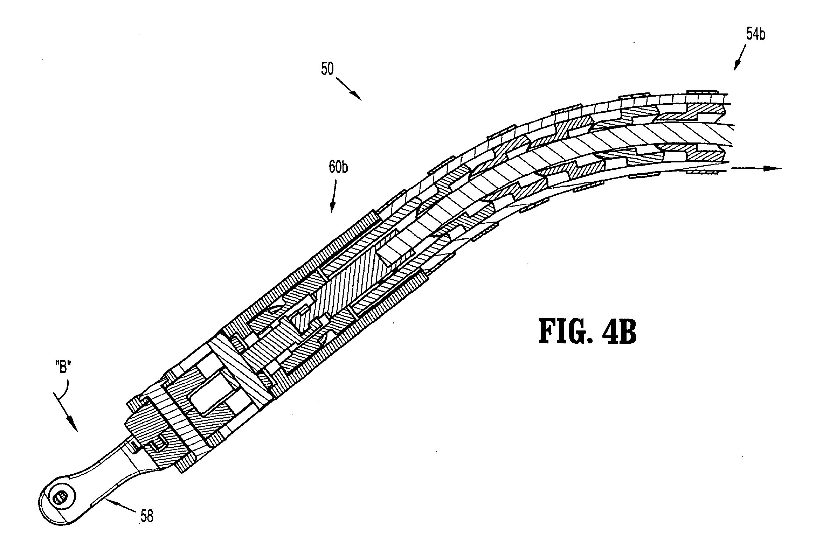

Referring now to FIG. 4, there is illustrated a surgical suturing apparatus 50 for use in transporting suture deployment system 10 through a cannula and manipulating surgical needle 13 through tissue. Surgical suturing apparatus 50 generally includes a body portion 52 having an elongate tubular member 54 extending distally from body portion 52. First and second jaws 56 and 58 are movably mounted on a distal end 60 of elongate tubular member 54. A pair of handles 66 and 68 are movably mounted on body portion 52 and are operable to move first and second jaws 56 and 58 between an open position substantially spaced apart to a closed position wherein first and second jaws 56 and 58 are substantially adjacent each other. First and second jaws 56 and 58 are provided to alternately engage surgical needle 14 and pass surgical needle 14 through tissue. Specifically, first and second jaws 56 and 58 include first and second needle receiving holes 62 and 64, respectively, for receipt of surgical needle 14. A toggle lever 70 is provided on body portion 52 and operates to alternately secure surgical needle 14 within needle holes 62 and 64 of first and second jaws 56 and 58, respectively. Engagement structure (not shown) is associated with toggle lever 70 and functions to alternately engage slots 46 and 48 in surgical needle 14 to alternately secure surgical needle 14 in first and second jaws 56 and 58. In this manner, surgical needle 14 can initially be secured and controlled within first jaw 56 and, upon closure of first and second jaws 56 and 58 and operation of toggle lever 70, control passed to second jaw 58 to enable surgical needle 14 to be secured therein. A loading lever 72 is provided on body portion 52 to enable surgical needle 14 to be loaded into first and second jaws 56 and 58. An exemplary example of surgical suturing apparatus 50 is disclosed in U.S. Patent No. 5,674,229 to Tovey et al. the entire disclosure of which is incorporated by reference herein.

As seen in FIG. 4A, surgical suturing apparatus 50 may include an elongate tubular member 54a having a distal end 60a that may be pivoted off-axis, in the direction of arrow "B" or opposite thereto, with respect to a longitudinal axis of tubular member 54a. It is further contemplated that distal end 60a may rotate about the longitudinal axis as indicated by arrows "C".

As seen in FIG. 4B, surgical suturing apparatus 50 may include an elongate tubular member 54b having a distal end 60b that may be articulated off-axis, about a plurality of joints or the like.

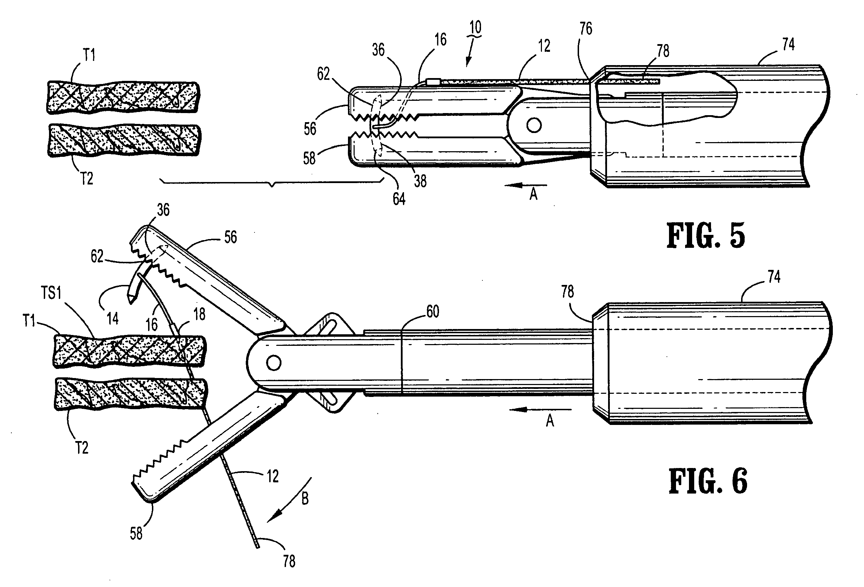

Referring now to FIGS. 4-9, and initially with regard to FIG. 5, the operation of surgical suturing apparatus 50 and suture deployment system 10 to insert target suture 12 into tissue will now be described. Surgical needle 14 is initially positioned within first and second jaws 56 and 58 of surgical suturing apparatus 50 such that first pointed end 36 of surgical needle 14 is located within first needle hole 62 in first jaw 56 and second pointed end 38 of surgical needle 14 is located within second needle hole 64 in second jaw 58. Toggle lever 70 (FIG. 4) is actuated to secure first pointed end 36 of surgical needle 14 within first needle hole 62 in first jaw 56. While not specifically shown, this is accomplished by advancing engagement structure associated with first jaw 56 and toggle lever 70 into engagement with engagement slot 46 adjacent first pointed and 36 of surgical needle 14. Second pointed end 38 of surgical needle 14 is free to move in and out of second needle hole 64 in second jaw 58.

During various surgical operations, a cannula, such as, for example, a cannula 74 is inserted into the body cavity to provide access for surgical suturing apparatus 50. As noted hereinabove, suture deployment system 10 allows target suture 12 to be transported through cannula 74 without causing undue bending of target suture 12. Handles 66 and 68 of surgical suturing apparatus 50 are actuated to move first and second jaws 56 and 58 to the closed position. As shown, as first and second jaws 56 and 58 are advanced in the direction of arrow A through cannula 74 and out a distal end 76 of cannula 74. Flexible suture 16 enables target suture 12 to remain relatively straight and lie along first and second jaws 56 and 58 as target suture 12 is passed through cannula 74. As best shown in FIGS. 5 and 6, as target suture 12 is advanced in the direction of arrow A, a proximal end 78 of target suture 12 passes through cannula 74 and out and distal end 76 of cannula 74.

Referring now to FIG. 6, as proximal end 78 of target suture 12 clears distal end 78 of cannula 74, target suture 12 is free to fall down alongside first and second jaws 56 and 58 in the direction of arrow B as shown. Specifically, the flaccid nature of flexible suture 16 allows target suture 12 to remain relatively straight while flexible suture 16 bends during manipulation of surgical needle 14. Once proximal end 78 of target suture 12 has cleared distal end 76 of cannula 74, handles 66 and 68 of surgical suturing apparatus 50 (FIG. 4) may be manipulated to move first and second jaws 56 and 58 to an open position spaced apart from each other. Thereafter, open first and second jaws 56 and 58 may be positioned about a pair of relatively tough tissue sections such as, for example, tissue sections T1 and T2 to be sutured together. As noted hereinabove, surgical needle 14 is secured within first jaw 56. At this stage, flexible suture 16 extends from surgical needle 14 and is secured to target suture 12 by collar 18 as described hereinabove. Target suture 12 is now located adjacent first and second tissue sections T1 and T2 while collar 18 is substantially adjacent first tissue surface TS1 of first tissue T1.

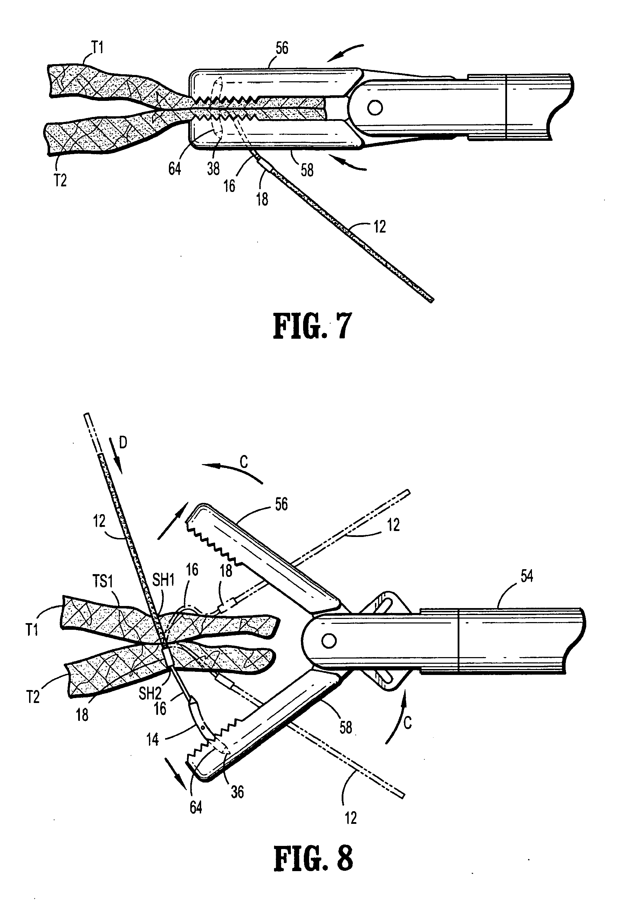

Referring to FIG. 7, once first and second jaws 56 and 58 have been properly positioned about first and second tissue sections T1 and T2, handles 66 and 68 are manipulated to bring first and second jaws 56 and 58 to a closed position adjacent each other. As first and second jaws 56 and 58 are moved to the closed position, surgical needle 14, and specifically, second pointed end 38 of surgical needle 14, pierces first and second tissue sections T1 and T2 and enters second needle hole 64 in second jaw 58. As shown, flexible suture 16 attached to target suture 12 by collar 18 is free to move along with the movement of surgical needle 14 without causing undue bending of target suture 12. Prior to opening first and second jaws 56 and 58, toggle lever 70 (FIG. 4) is operated to pass control of surgical needle 14 from first jaw 56 to second jaw 58. Specifically, surgical needle 14 is released from engagement with first jaw 56 and engagement structure associated with second jaw 58 engages second engagement slot 48 in surgical needle 14. Thus, surgical needle 14 is securely engaged within second hole 64 in second jaw 58.

As best shown in FIG. 8, once tissue sections T1 and2 have been pierced by surgical needle 14, handles 66 and 68 may be manipulated to move first and second jaws 56 and 58 to the open position spaced apart from each other. As shown, as first and second jaws 56 and 58 are moved to the open position, surgical needle 14 is drawn through first and second suture holes SH1 and SH2 formed through first and second tissue sections T1 and T2. Flexible suture 16 is also drawn through first and second suture holes SH1 and SH2. As flexible suture 16 is drawn through first and second suture holes SH1 and SH2, collar 18, fixed to flexible suture 16, is drawn along first tissue surface TS1 I until collar 18 is located adjacent first suture hole SH1 in first tissue T1. Target suture 12, being relatively stiff in nature, tends to rise up from alongside first and second tissue sections T1 and T2 in the direction of arrow C. When collar 18 begins to be drawn through first suture hole SH1, relatively stiff target suture 12 "flips up" in the direction of arrow C and generally assumes a substantially perpendicular orientation relative to first tissue surface TS1. As surgical instrument 50 is further manipulated, second jaw 58 draws surgical needle 14, flexible suture 16, collar 18 and target suture 12 through first and second suture holes SH1 and SH2 in the direction of arrow D.

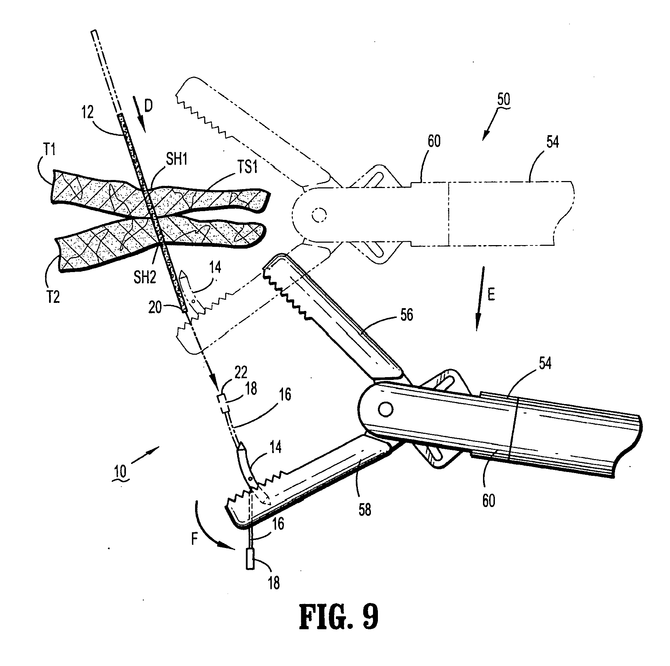

Referring now to FIG. 9, once target suture 12 has been pulled through first and second suture holes SH1 and SH2 in first and second tissue sections T1 and T2, respectively, distal end 60 of elongate tubular member 54 is moved in the direction of arrow E to move second jaw 58 downwardly relative to first and second tissue sections T1 and T2 thereby drawing target suture 12 completely through first and second tissue sections T1 and T2 in the direction of arrow D. After target suture 12 has been fully seated within first and second tissue sections T1 and T2, further movement of second jaw 58 away from tissue sections T1 and T2 exerts further tension on surgical needle 14, flexible suture 16 and collar 18. Once a predetermined amount of tension has been achieved, distal end 20 of target suture 12 pulls free of proximal end 22 of collar 18 thereby releasing target suture 12 from the remainder of suture deployment system 10. Barbs 30 on outer surface 32 of target suture 12 facilitate securing target suture 12 within first and second tissue sections T1 and T2. The remainder of suture deployment system 10, including flexible suture 16 and collar 18 are free to fall down in the direction of arrow F alongside second jaw 58.

In this manner, suture deployment system 10 allows relatively stiff target suture 12 to be manipulated through cannula 74 and through tissue sections T1 and T2 without risk of bending or breaking target suture 12.

It will be understood that various modifications may be made to the embodiments disclosed herein. For example, various other types of target sutures may be deployed utilizing the disclosed suture deployment system. Further, the disclose suture deployment system may be provided with a variety of other types of surgical needles such as single pointed, straight, curved, etc. Additionally, other releasable means for securing the flexible suture to the target suture may be provided, such as, for example, adhesives, interlocking braids, welding, etc. Therefore, the above description should not be construed as limiting, but merely as exemplifications of particular embodiments. Those skilled in the art will envision other modifications within the scope and spirit of the claims appended hereto.
The invention may be described by reference to the following numbered paragraph:-
1. A suture deployment system comprising:
   a surgical needle;
   a flexible suture having a distal end and a proximal end, the distal end of the flexible suture being attached to the surgical needle; and
   a target suture having a distal end and a proximal end, wherein the distal end of the target suture is secured to the proximal end of the flexible suture, and wherein at least one of the flexible suture and the target suture comprise a shape memory polymer.
2. The suture deployment system as recited in paragraph 1, wherein the target suture includes a plurality of barbs located on an outer surface of the target suture.
3. The suture deployment system as recited in paragraph 2, wherein the plurality of barbs comprise a shape memory polymer.
4. The suture deployment system as recited in paragraph 1, further comprising a hollow collar having a distal end and a proximal end, the hollow collar being positioned intermediate the proximal end of the flexible suture and the distal end of the target suture.
5. The suture deployment system of paragraph 1, wherein the shape memory polymer is selected from the group consisting of bioabsorbable materials, non-degradable materials and combinations thereof.
6. The suture deployment system of paragraph 1, wherein the shape memory polymer comprises a non-degradable material selected from the group consisting of polyolefins, polyethylene glycols, polyethylene oxides, polyolefin copolymers, fluorinated polyolefins, polyamides, polyamines, polyimines, polyesters, polyethers, polybutesters, polyurethanes, acrylic polymers, methacrylics polymers, vinyl halide polymers and copolymers, polyvinyl alcohols, polyvinyl ethers, polyvinylidene halides, polychlorofluoroethylene, polyacrylonitrile, polyaryletherketones, polyvinyl ketones, polyvinyl aromatics, polyvinyl esters, copolymers of vinyl monomers, acrylonitrile-styrene copolymers, ABS resins, ethylene-vinyl acetate copolymers, alkyd resins, polycarbonates, polyoxymethylenes, polyphosphazines, polyimides, epoxy resins, aramids, rayons, spandex, silicones, and combinations thereof.
7. The suture deployment system of paragraph 1, wherein the shape memory polymer comprises a bioabsorbable material selected from the group consisting of aliphatic polyesters, polyamides, polyamines, polyalkylene oxalates, poly(anhydrides), polyamidoesters, copoly(ether-esters), poly(carbonates), poly(hydroxyalkanoates), polyimide carbonates, poly(imino carbonates), polyorthoesters, polyoxaesters, polyphosphazenes, poly (propylene fumarates), polyurethanes, polymer drugs, biologically modified bioabsorbable polymers, and copolymers, homopolymers, and combinations thereof.
8. The suture deployment system of paragraph 7, wherein the shape memory polymer comprises an aliphatic polyester selected from the group consisting of homopolymers and copolymers of lactide, glycolide, epsilon-caprolactone, p-dioxanone, trimethylene carbonate, alkyl derivatives of trimethylene carbonate, Δ-valerolactone, β-butyrolactone, γ-butyrolactone, ε-decalactone, hydroxybutyrate, hydroxyvalerate, 1,4-dioxepan-2-one, 1,5-dioxepan-2-one, 6,6-dimethyl-1,4-dioxan-2-one, 2,5-diketomorpholine, pivalolactone, α,α diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one, and combinations thereof.
9. The suture deployment system of paragraph 1, wherein the shape memory polymer comprises a biodegradable polymer selected from the group consisting of poly(amino acids), collagen, elastin, fibrin, fibrinogen, silk, albumin, peptides including sequences for laminin and fibronectin, hyaluronic acid, dextran, alginate, chitin, chitosan, cellulose, glycosaminoglycan, gut, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulfate sodium salt, nitrocelluloses, chitosan, and combinations thereof.
10. The suture deployment system of paragraph 1, wherein the shape memory polymer comprises a polymer selected from the group consisting of oligo (epsilon-caprolactone) dimethacrylates, oligo (epsilon-caprolactone) butyl acrylates, (n-butyl acrylate), oligo (epsilon caprolactone) diol/oligo (p-dioxanone) diol copolymers, polycaprolactone dimethacrylate poly(butyl acrylate) blends, and combinations thereof.
11. The suture deployment system of paragraph 1, wherein the shape memory polymer comprises a block copolymer of polydioxanone and polylactide.
12. The suture deployment system of paragraph 11, wherein the polydioxanone is present in an amount from about 5 mol% to about 20 mol% of the copolymer and the polylactide is present in an amount from about 80 mol% to about 95 mol% of the copolymer.
13. The suture deployment system of paragraph 1, wherein the shape memory polymer comprises a block copolymer of trimethylene carbonate and polylactide.
14. The suture deployment system of paragraph 13, wherein the trimethylene carbonate is present in an amount from about 5 mol% to about 20 mol% of the copolymer and the polylactide is present in an amount from about 80 mol% to about 95 mol% of the copolymer.
15. A suture deployment system comprising:
   a surgical needle having a body portion and a tissue penetrating tip formed at each end of the body portion;
   a multifilament suture having a distal end and a proximal end, the distal end of the multifilament suture being secured within the suture hole in the body portion of the surgical needle;
   a hollow collar having a distal end and a proximal end, the proximal end of the multifilament suture being within the distal end of the hollow collar; and
   a monofilament target suture having a distal end and a proximal end, wherein the distal end of the monofilament target suture is releasably secured within the proximal end of the hollow collar, and wherein at least one of the multifilament suture and monofilament target suture comprise a shape memory material.
16. The suture deployment system as recited in paragraph 15, wherein the monofilament target suture is a barbed suture.
17. The suture deployment system as recited in paragraph 15, wherein the surgical needle includes at least one crimping bump adjacent the suture hole.
18. The suture deployment system as recited in paragraph 15, wherein the surgical needle includes at least one engagement slot for receipt of engagement structure associated with a surgical suturing apparatus.
19. The suture deployment system as recited in paragraph 15, wherein the barbed suture is a compound barbed suture including:
   an elongated body; and
   at least one barb extending from the elongated body and defining an inner surface, the inner surface including a first portion disposed at a first orientation relative to a longitudinal axis of the elongated body and a second portion disposed at a second orientation relative to the longitudinal axis.
20. The suture deployment system as recited in paragraph 19, wherein at least one of the first and second portions is substantially linear.
21. The suture deployment system as recited in paragraph 20, wherein the first and second portions are at first and second angles relative to respective longitudinal axes of the elongated body.
22. The suture deployment system as recited in paragraph 21, wherein the second angle is less than the first angle.
23. The suture deployment system as recited in paragraph 21, wherein the first angle is between about 0 degrees to about 90 degrees.
24. The suture deployment system as recited in paragraph 21, wherein the second angle is between about 0 degrees to about 90 degrees.

## Claims

1. A suture deployment system comprising:
a surgical needle;
a flexible suture having a distal end and a proximal end, the distal end of the flexible suture being attached to the surgical needle; and
a target suture having a distal end and a proximal end, wherein the distal end of the target suture is secured to the proximal end of the flexible suture, and wherein at least one of the flexible suture and the target suture comprise a shape memory polymer.

2. The suture deployment system as recited in claim 1, wherein the target suture includes a plurality of barbs located on an outer surface of the target suture.

3. The suture deployment system as recited in claim 2, wherein the plurality of barbs comprise a shape memory polymer.

4. The suture deployment system as recited in any preceding claim, further comprising a hollow collar having a distal end and a proximal end, the hollow collar being positioned intermediate the proximal end of the flexible suture and the distal end of the target suture.

5. The suture deployment system of any preceding claim, wherein the shape memory polymer is selected from the group consisting of bioabsorbable materials, non-degradable materials and combinations thereof.

6. The suture deployment system of claim 5, wherein the shape memory polymer comprises a non-degradable material selected from the group consisting of polyolefins, polyethylene glycols, polyethylene oxides, polyolefin copolymers, fluorinated polyolefins, polyamides, polyamines, polyimines, polyesters, polyethers, polybutesters, polyurethanes, acrylic polymers, methacrylics polymers, vinyl halide polymers and copolymers, polyvinyl alcohols, polyvinyl ethers, polyvinylidene halides, polychlorofluoroethylene, polyacrylonitrile, polyaryletherketones, polyvinyl ketones, polyvinyl aromatics, polyvinyl esters, copolymers of vinyl monomers, acrylonitrile-styrene copolymers, ABS resins, ethylene-vinyl acetate copolymers, alkyd resins, polycarbonates, polyoxymethylenes, polyphosphazines, polyimides, epoxy resins, aramids, rayons, spandex, silicones, and combinations thereof.

7. The suture deployment system of claim 5, wherein the shape memory polymer comprises a bioabsorbable material selected from the group consisting of aliphatic polyesters, polyamides, polyamines, polyalkylene oxalates, poly(anhydrides), polyamidoesters, copoly(ether-esters), poly(carbonates), poly(hydroxyalkanoates), polyimide carbonates, poly(imino carbonates), polyorthoesters, polyoxaesters, polyphosphazenes, poly (propylene fumarates), polyurethanes, polymer drugs, biologically modified bioabsorbable polymers, and copolymers, homopolymers, and combinations thereof.

8. The suture deployment system of claim 5, wherein the shape memory polymer comprises an aliphatic polyester selected from the group consisting of homopolymers and copolymers of lactide, glycolide, epsilon-caprolactone, p-dioxanone, trimethylene carbonate, alkyl derivatives of trimethylene carbonate, Δ-valerolactone, β-butyrolactone, γ-butyrolactone, ε-decalactone, hydroxybutyrate, hydroxyvalerate, 1,4-dioxepan-2-one, 1,5-dioxepan-2-one, 6,6-dimethyl-1,4-dioxan-2-one, 2,5-diketomorpholine, pivalolactone, α,α diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one, and combinations thereof.

9. The suture deployment system of claim 5, wherein the shape memory polymer comprises a biodegradable polymer selected from the group consisting of poly(amino acids), collagen, elastin, fibrin, fibrinogen, silk, albumin, peptides including sequences for laminin and fibronectin, hyaluronic acid, dextran, alginate, chitin, chitosan, cellulose, glycosaminoglycan, gut, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulfate sodium salt, nitrocelluloses, chitosan, and combinations thereof.

10. The suture deployment system of claim 5, wherein the shape memory polymer comprises a polymer selected from the group consisting of oligo (epsilon-caprolactone) dimethacrylates, oligo (epsilon-caprolactone) butyl acrylates, (n-butyl acrylate), oligo (epsilon caprolactone) diol/oligo (p-dioxanone) diol copolymers, polycaprolactone dimethacrylate poly(butyl acrylate) blends, and combinations thereof.

11. The suture deployment system of claim 5, wherein the shape memory polymer comprises a block copolymer of polydioxanone and polylactide; preferably wherein the polydioxanone is present in an amount from about 5 mol% to about 20 mol% of the copolymer and the polylactide is present in an amount from about 80 mol% to about 95 mol% of the copolymer.

12. The suture deployment system of claim 5, wherein the shape memory polymer comprises a block copolymer of trimethylene carbonate and polylactide; preferably wherein the trimethylene carbonate is present in an amount from about 5 mol% to about 20 mol% of the copolymer and the polylactide is present in an amount from about 80 mol% to about 95 mol% of the copolymer.

13. A suture deployment system comprising:
a surgical needle having a body portion and a tissue penetrating tip formed at each end of the body portion;
a multifilament suture having a distal end and a proximal end, the distal end of the multifilament suture being secured within the suture hole in the body portion of the surgical needle;
a hollow collar having a distal end and a proximal end, the proximal end of the multifilament suture being within the distal end of the hollow collar; and
a monofilament target suture having a distal end and a proximal end, wherein the distal end of the monofilament target suture is releasably secured within the proximal end of the hollow collar, and wherein at least one of the multifilament suture and monofilament target suture comprise a shape memory material.

14. The suture deployment system as recited in claim 13, wherein the monofilament target suture is a barbed suture.

15. The suture deployment system as recited in claim 14, wherein the surgical needle includes at least one crimping bump adjacent the suture hole.

16. The suture deployment system as recited in any of claims 13 to 15, wherein the surgical needle includes at least one engagement slot for receipt of engagement structure associated with a surgical suturing apparatus.

17. The suture deployment system as recited in any of claims 13 to 16, wherein the barbed suture is a compound barbed suture including:
an elongated body; and
at least one barb extending from the elongated body and defining an inner surface, the inner surface including a first portion disposed at a first orientation relative to a longitudinal axis of the elongated body and a second portion disposed at a second orientation relative to the longitudinal axis.

18. The suture deployment system as recited in claim 17, wherein at least one of the first and second portions is substantially linear.

19. The suture deployment system as recited in claim 17, wherein the first and second portions are at first and second angles relative to respective longitudinal axes of the elongated body; preferably wherein the second angle is less than the first angle.

20. The suture deployment system as recited in claim 19, wherein the first angle is between about 0 degrees to about 90 degrees; and/or wherein the second angle is between about 0 degrees to about 90 degrees.
